(19) Europäisches Patentamt
European Patent Office
Office européen des brevets

(11) EP 1 058 695 B1

(12) EUROPÄISCHE PATENTSCHRIFT

(45) Veröffentlichungstag und Bekanntmachung des Hinweises auf die Patenterteilung:
**06.08.2003 Patentblatt 2003/32**

(21) Anmeldenummer: **99907562.5**

(22) Anmeldetag: **24.02.1999**

(51) Int Cl.7: **C08F 8/30**, A61L 15/00

(86) Internationale Anmeldenummer:
**PCT/EP99/01188**

(87) Internationale Veröffentlichungsnummer:
**WO 99/043720 (02.09.1999 Gazette 1999/35)**

# (54) VERFAHREN ZUR VERNETZUNG VON HYDROGELEN MIT BIS- UND POLY-2-OXAZOLIDINONEN

METHOD FOR CROSS-LINKING HYDROGELS WITH BIS- AND POLY-2-OXAZOLIDINONES

PROCEDE POUR RETICULER DES HYDROGELS AVEC DES BIS- ET POLY-2-OXAZOLIDINONES

(84) Benannte Vertragsstaaten:
**BE DE ES FI FR GB IT NL SE**

(30) Priorität: **26.02.1998 DE 19807992**

(43) Veröffentlichungstag der Anmeldung:
**13.12.2000 Patentblatt 2000/50**

(73) Patentinhaber: **BASF AKTIENGESELLSCHAFT
67056 Ludwigshafen (DE)**

(72) Erfinder:
- **FUNK, Rüdiger
D-65527 Niedernhausen (DE)**
- **FRENZ, Volker
D-55246 Mainz-Kostheim (DE)**
- **RIEGEL, Ulrich
D-60384 Frankfurt am Main (DE)**
- **WEISMANTEL, Matthias
D-63637 Jossgrund-Oberndorf (DE)**
- **ENGELHARDT, Fritz
Chesapeake, VA 23320 (US)**
- **DANIEL, Thomas
Chesapeake, VA 23321 (US)**

(56) Entgegenhaltungen:

| | |
|---|---|
| **EP-A- 0 260 011** | **WO-A-94/09043** |
| **FR-A- 1 455 783** | **FR-A- 2 525 121** |
| **GB-A- 642 453** | **US-A- 3 364 181** |
| **US-A- 3 448 063** | **US-A- 3 758 629** |
| **US-A- 4 056 502** | **US-A- 4 123 419** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 058 695 B1

Printed by Jouve, 75001 PARIS (FR)

## Beschreibung

Die vorliegende Erfindung betrifft ein Verfahren zur Gel- bzw. Oberflächennachvernetzung von wasserabsorbierenden Hydrogelen durch Einpolymerisieren von Bis- und Poly-2-oxazolidinonen.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wäßrigen Flüssigkeiten quellbare Naturprodukte, wie beispielsweise Guarderivate. Solche Hydrogele werden als wäßrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie z.B. Rewet in der Windel und AUL, werden hydrophile, hochquellfähige Hydrogele im allgemeinen oberflächen- oder gelnachvernetzt. Diese Nachvernetzung ist dem Fachmann an sich bekannt und erfolgt bevorzugt in wäßriger Gelphase oder als Oberflächennachvernetzung der gemahlenen und abgesiebten Polymerpartikel.

Dazu geeignete Vernetzer sind Verbindungen, die mindestens zwei Gruppen enthalten, die mit den Carboxylgruppen des hydrophilen Polymeren kovalente Bindungen bilden können. Geeignete Vernetzer sind beispielsweise Di- oder Polyglycidylverbindungen, wie Phosphonsäurediglycidylester, Alkoxysilylverbindungen, Polyaziridine, Polyamine oder Polyamidoamine, wobei die genannten Verbindungen auch in Mischungen untereinander verwendet werden können (siehe beispielsweise EP-A-0 083 022, EP-A-0 543 303 und EP-A-0 530 438). Als Vernetzer geeignete Polyamidoamine sind insbesondere in der EP-A-0 349 935 beschrieben.

Ein wesentlicher Nachteil dieser Vernetzer ist deren hohe Reaktivität. Diese ist zwar, was den chemischen Umsatz betrifft, erwünscht, birgt aber ein höheres toxikologisches Potential. Die Verarbeitung derartiger Vernetzer im Produktionsbetrieb erfordert besondere Schutzvorkehrungen, um den Anforderungen der geltenden Sicherheitsaustimmungen und der Arbeitsplatzhygiene gerecht zu werden. Darüber hinaus erscheint die Verwendung derartig modifizierter Polymere in Hygieneartikeln bedenklich.

Als Vernetzer sind auch polyfunktionelle Alkohole bekannt. Beispielsweise lehren EP-A-0 372 981, US-A-4 666 983 sowie US-A-5 385 983 die Verwendung von hydrophilen Polyalkoholen bzw. die Verwendung von Polyhydroxytensiden. Die Reaktion wird hiernach bei Temperaturen von 120-250°C durchgeführt. Das Verfahren hat den Nachteil, daß die zur Vernetzung führende Veresterungsreaktion selbst bei diesen Temperaturen nur relativ langsam abläuft.

Somit bestand die Aufgabe darin, unter Verwendung relativ reaktionsträger, aber dennoch mit Carboxylgruppen reaktionsfähiger Verbindungen eine im Vergleich zum Stand der Technik ebenso gute oder bessere Gel- bzw. Oberflächennachvernetzung zu erreichen. Diese Aufgabe war so zu lösen, daß die Reaktionszeit möglichst kurz und die Reaktionstemperatur möglichst niedrig sind. Im Idealfall sollten dieselben Reaktionsbedingungen herrschen wie bei der Verwendung von hochreaktiven Epoxiden.

Überraschenderweise wurde nun gefunden, daß Bis- und Poly-2-Oxazolidinone hervorragend zur Lösung dieser Aufgabe geeignet sind. Insbesondere kann die Reaktivität dieser Vernetzer durch Zugabe von anorganischen oder organischen sauren Katalysatoren gesteigert werden. Als Katalysatoren geeignet sind die bekannten anorganischen Mineralsäuren, deren saure Salze mit Alkalimetallen oder Ammonium, sowie deren Anhydride. Geeignete organische Katalysatoren sind die bekannten Carbonsäuren, Sulfonsäuren sowie Aminosäuren.

Gegenstand der Erfindung ist ein Verfahren zur Oberflächennachvernetzung wasserabsorbierender Polymere, dadurch gekennzeichnet, daß die Polymere mit einer Oberflächennachvernetzungslösung be handelt und während oder nach dem Behandeln durch Temperaturerhöhung nachvernetzt und getrocknet werden, dadurch gekennzeichnet, daß der Vernetzer ein Bis-2-Oxazolidinon oder ein Poly-2-Oxazolidinon, enthaltend Struktureinheiten der allgemeinen Formel

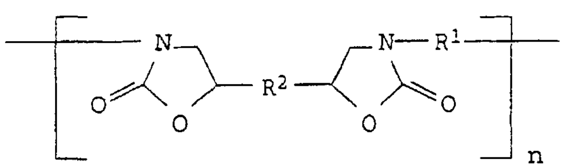

(1),

worin

R1 verzweigtes oder unverzweigtes $C_1$-$C_{18}$-Alkylen, verzweigtes oder unverzweigtes $C_2$-$C_{18}$-Alkenylen, $C_5$-$C_8$-Cycloalkylen, Phenylen, Naphthylen, Anthracenylen, mit Kohlenwasserstoffen substituiertes Phenylen, Naphthylen oder Anthracenylen oder ein anderer substituierter oder unsubstituierter $C_6$-$C_{18}$-Arylenrest,

$R^2$ verzweigtes oder unverzweigtes $C_1$-$C_{18}$-Alkylen und

n eine ganze Zahl von 1 bis 50 bedeuten,

oder ein Gemisch von Bis-2-Oxazolidinonen und Poly-2-Oxazolidinonen gelöst in einem inerten Lösemittel enthält.

Steht $R^1$ für einen Alkylen- oder Alkenylenrest, so bedeutet es vorzugsweise einen solchen Rest mit einer Kettenlänge von 3 bis 12, insbesondere 5 bis 10 Kohlenstoffatomen. $R^2$ bedeutet vorzugsweise einen Alkylenrest mit einer Kettenlänge von 3 bis 12, insbesondere 5 bis 10 Kohlenstoffatomen.

Endständige Struktureinheiten der Formel 1 sind endgruppenverschlossen. Als Endgruppe kann jeder Rest verwendet werden, der in die Bis-2-Oxazolidinone oder Poly-2-Oxazolidinone eingeführt werden kann, und der an diesen Verbindungen chemisch stabil ist. Geeignete Reste, mit denen die Struktureinheiten der Formel 1 endgruppenverschlossen werden können, sind beispielsweise Wasserstoff, verzweigtes oder unverzweigtes $C_1$-$C_{18}$-Alkyl, verzweigtes oder unverzweigtes $C_2$-$C_{18}$-Alkenyl, Phenyl, Naphthyl, Anthracenyl, mit Kohlenwasserstoffen substituiertes Phenyl, Naphthyl oder Anthracenyl oder ein anderer substituierter oder unsubstituierter $C_6$-$C_{18}$-Arylrest.

Bevorzugt enthalten die Poly-2-oxazolidinone n Bis-2-oxazolidinoneinheiten.

n bedeutet vorzugsweise eine Zahl zwischen 1 und 10, besonders bevorzugt zwischen 3 und 6. Die Temperatur zur Nachvernetzung beträgt vorzugsweise zwischen 50 und 250°C, insbesondere 50-200°C, speziell zwischen 100-180°C.

Zur Beschleunigung der Oberflächennachvernetzungsreaktion kann der Reaktionsmischung ein saurer Katalysator zugesetzt werden. Als Katalysator im erfindungsgemäßen Verfahren sind alle anorganischen Säuren, deren korrespondierende Anhydride, bzw. organischen Säuren und deren korrespondierende Anhydride verwendbar. Beispiele sind Borsäure, Schwefelsäure, Iodwasserstoffsaure. Phosphorsaure, Weinsäure, Essigsaure und Toluolsulfonsaure. Insbesondere sind auch deren polymere Formen, Anhydride, sowie die sauren Salze, wie sie z.B. bei den mehrwertigen Säuren auftreten, geeignet. Beispiele hierfür sind Boroxid, Schwefeltrioxid, Diphosphorpentaoxid, und Ammoniumdihydrogenphosphat.

Das erfindungsgemäße Verfahren wird vorzugsweise so durchgeführt, daß eine Lösung des Oberflächennachvernetzers auf das trockene Grundpolymerpulver aufgesprüht wird. Im Anschluß an das Aufsprühen wird das Polymerpulver thermisch getrocknet, wobei die Vernetzungsreaktion sowohl vor als auch während der Trocknung stattfinden kann. Bevorzugt ist das Aufsprühen einer Lösung des Vernetzers in Reaktionsmischern und Sprühmischern oder Misch- und Trocknungsanlagen wie beispielsweise Lödige-Mischer, ®BEPEX-Mischer, ®NAUTA-Mischer, ®SHUGGI-Mischer oder ®PROCESSALL. Überdies können auch Wirbelschichttrockner eingesetzt werden. Die Trocknung kann im Mischer selbst erfolgen, durch Beheizung des Mantels oder Einblasen von Warmluft. Ebenso geeignet ist ein nachgeschalteter Trockner wie z.B. ein Hordentrockner, ein Drehrohrofen, oder eine beheizbare Schnecke. Es kann aber auch beispielsweise eine azeotrope Destillation als Trocknungsverfahren benutzt werden. Die Verweilzeit bei der bevorzugten Temperatur im Reaktionsmischer oder Trockner liegt bei 5 bis 90 Minuten, bevorzugt bei weniger als 30 Minuten, ganz besonders bevorzugt bei weniger als 10 Minuten.

Als inertes Lösemittel bevorzugt ist Wasser sowie Gemische von Wasser mit einfachen oder mehrfachfunktionellen Alkoholen. Es können jedoch alle mit Wasser unbegrenzt mischbaren organischen Lösemittel, wie beispielsweise bestimmte Ester und Ketone eingesetzt werden, die nicht selbst unter den Verfahrensbedingungen reaktiv sind. Sofern ein Alkohol/Wasser-Gemisch eingesetzt wird, beträgt der Alkoholgehalt dieser Lösung 10-90 Gew.-%, bevorzugt 30-70 Gew.-%, insbesondere 40-60 Gew.-%. Es können alle mit Wasser unbeschränkt mischbaren Alkohole eingesetzt werden sowie Gemische mehrerer Alkohole (z.B. Methanol + Glycerin + Wasser). Besonders bevorzugt ist die Verwendung folgender Alkohole in wäßriger Lösung: Methanol, Ethanol, Isopropanol, Ethylenglykol und besonders bevorzugt 1,2-Propandiol sowie 1,3-Propandiol. Die Oberflächennachvernetzungslösung wird in einem Verhältnis von 1-20 Gew.-% bezogen auf die Polymermase eingesetzt. Besonders bevorzugt ist eine Losungsmenge von 2,5-15 Gew.-% bezüglich Polymer. Der Vernetzer selbst wird dabei in einer Menge von 0,01-1,0 Gew.-% bezogen auf das eingesetzte Polymer verwendet.

Das wasserabsorbierende Polymer ist bevorzugt eine polymere Acrylsaure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere enthalten (0,001-10 mol-%), ganz besonders bevorzugt sind jedoch Polymere die über radikalische Polymerisation erhalten wurden und bei denen ein mehrfunktioneller ethylenisch ungesättigter Radikalvernetzer verwendet wurde, der zusätzlich noch mindestens eine freie Hydroxylgruppe trägt (wie z.B. Pentaerythritoltriallylether oder Trimethylolpropandiallylether).

Die im erfindungsgemäßen Verfahren einzusetzenden hydrophilen, hochquellfähigen Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether oder in wäßrigen Flüssigkeiten quellbare Naturprodukte, wie beispielsweise Guarderivate. Diese Hydrogele sind dem Fachmann bekannt und beispielsweise beschrieben in US-A-4 286 082, DE-C-27 06 135, US-A-4 340 706, DE-C-37 13 601, DE-C-28 40 010, DE-A-43 44 548, DE-A-40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A-26 12 846, DE-A-40 20 780 EP-A-C 205 674, US-A-5 145 906, EP-A-0 530 438, EP-A-0 670 073, US-A-4 057 521, US-A-4 062 817, US-A-4 525 527, US-A-4 295 987, US-A-5 011 892, US-A-4 076 663 oder US-A-4 931 497. Der Inhalt der vorstehend genannten Patentdokumente ist ausdrücklich Bestandteil der vorliegenden Offenbarung.

Zur Herstellung dieser hydrophilen, hochquellfähigen Hydrogele geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acryiamido-2-methylpropansulfonsäure, 2-Acrylamido-2-methylpropanphosphonsäure sowie deren Salze z.B. Natrium-, Kalium- cder Ammoniumsalz, deren Amide, Hydroxyalkylester und aminogruppen- oder ammoniumgruppenhaltige Ester und Amide. Des weiteren sind wasserlösliche N-Vinylamide oder auch Diallyldimethylammoniumchlorid geeignet. Bevorzugte hydrophile Monomere sind Verbindungen der allgemeinen Formel 2

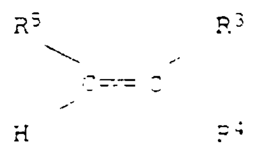

(2),

worin

$R^3$ Wasserstoff, Methyl oder Ethyl,

$R^4$ -COOR$^6$, eine Sulfonylgruppe, eine Phosphonylgruppe, eine mit ($C_1$-$C_4$)-Alkanol veresterte Phosphonylgruppe oder eine Gruppe der allgemeinen Formel 3

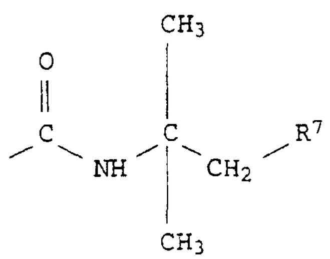

(3),

$R^5$ Wasserstoff, Methyl, Ethyl oder eine Carboxylgruppe,

$R^6$ Wasserstoff, Amino-($C_1$-$C_4$) -alkyl oder Hydroxy-($C_1$-$C_4$)-alkyl, Alkalimetall- oder Ammoniumion und

$R^7$ eine Sulfonylgruppe, eine Phosphonylgruppe oder eine Carboxylgruppe oder Alkalimetall- oder Ammoniumsalze dieser Gruppen, bedeuten.

Beispiele für ($C_1$-$C_4$)-Alkanole sind Methanol, Ethanol, n-Propanol oder n-Butanol. Besonders bevorzugte hydrophile Monomere sind Acrylsäure und Methacrylsäure sowie deren Alkalimetall- oder Ammoniumsalze, z.B. Natrium-, Kalium- und Ammoniumacrylat.

Geeignete Pfropfgrundlagen für hydrophile Hydrogele, die durch Pfropfcopolymerisation olefinisch ungesättigter Säuren oder ihrer Alkalimetall- oder Ammoniumsalze erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Geeignete Polyalkylenoxide haben beispielsweise die allgemeine Formel 4

$$R^8 - O - (CH_2 - \overset{\overset{\displaystyle X}{\vdots}}{CH} - O)_n - R^9 \qquad (4)$$

worin

| | |
|---|---|
| $R^8$ und $R^9$ | unabhängig voneinander Wasserstoff, Alkyl, Alkenyl oder Acyl, |
| X | Wasserstoff oder Methyl und |
| n | eine ganze Zahl von 1 bis 10 000 bedeuten. |

$R^8$ und $R^9$ bedeuten bevorzugt Wasserstoff, ($C_1$-$C_4$)-Alkyl, ($C_2$-$C_6$)-Alkenyl oder Phenyl. Bevorzugte Hydrogele sind insbesondere Polyacrylate, Polymethacrylate sowie die in US-A-4 931 497, US-A-5 011 892 und US-A-5 041 496 beschriebene Pfropfcopolymere.

Die hydrophilen, hochquellfähigen Hydrogele sind bevorzugt vernetzt, d.h. sie enthalten Verbindungen mit mindestens zwei Doppelbindungen, die in das Polymernetzwerk einpolymerisiert sind. Geeignete Vernetzer sind insbesondere Methylenbisacryl- bzw. -methacrylamid, Ester ungesättigter Mono- oder Polycarbonsäuren von Polyolen, wie Diacrylat oder Triacrylat, z.B. Butandiol- oder Ethylenglykoldiacrylat bzw. -methacrylat sowie Trimethylolpropantriacrylat und Allylverbindungen wie Allyl(meth)-acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in der EP-A-0 343 427 beschrieben sind. Besonders bevorzugt werden im erfindungsgemäßen Verfahren jedoch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Monoethylenglykoldiallylether, Glyceroldi- und triallylether, Polyallylether auf Basis Sorbitol, sowie alkoxylierte Varianten davon.

Die hydrophilen, hochquellfähigen Hydrogele können durch an sich bekannte Polymerisationsverfahren hergestellt werden. Bevorzugt ist die Polymerisation in wäßriger Lösung nach dem Verfahren der sogenannten Gelpolymerisation. Dabei werden 15 bis 50 Gew.-%ige wäßrige Lösungen eines oder mehrerer hydrophiler Monomerer und gegebenenfalls einer geeigneten Pfropfgrundlage in Gegenwart eines Radikalinitiators, vorzugsweise ohne mechanische Durchmischung, unter Ausnutzung des Trommsdorff-Norrish-Effektes (Makromol. Chem. 1, 169 (1947)), polymerisiert.

Die Polymerisationsreaktion kann im Temperaturbereich zwischen 0°C und 150°C, vorzugsweise zwischen 10°C und 100°C, sowohl bei Normaldruck als auch unter erhöhtem oder erniedrigtem Druck durchgeführt werden. Die Polymerisation kann auch in einer Schutzgasatmosphäre, vorzugsweise unter Stickstoff, ausgeführt werden.

Zur Auslösung der Polymerisation können energiereiche elektromagnetische Strahlen oder die üblichen chemischen Polymerisationsinitiatoren herangezogen werden. Solche sind beispielsweise organische Peroxide, wie Benzoylperoxid, tert. -Butylhydroperoxid, Methylethylketonperoxid, Cumolhydroperoxid, Azoverbindungen wie Azodiisobutyronitril sowie anorganische Peroxiverbindungen wie $(NH_4)_2S_2O_8$, $K_2S_2O_8$ oder $H_2O_2$. Sie können gegebenenfalls in Kombination mit Reduktionsmitteln wie Natriumhydrogensulfit, Eisen(II)-sulfat oder Redoxsystemen verwendet werden. Redoxsysteme enthalten als reduzierende Komponente im allgemeinen eine aliphatische und aromatische Sulfinsäure, wie Benzolsulfinsäure oder Toluolsulfinsäure oder Derivate dieser Säuren, wie Mannichaddukte aus Sulfinsäure, Aldehyden und Aminoverbindungen, wie sie in DE-C-13 01 566 beschrieben sind.

Durch mehrstündiges Nachheizen der Polymerisatgele im Temperaturbereich 50 bis 130°C, vorzugsweise 70 bis 100°C, können die Qualitätseigenschaften der Polymerisate noch verbessert werden.

Die erhaltenen Gele werden zu 0-100 mol-% bezogen auf eingesetztes Monomer neutralisiert, bevorzugt zu 25-100 mol-%, und besonders bevorzugt zu 50-85 mol-%, wobei die üblichen Neutralisationsmittel verwendet werden können, bevorzugt Alkalimetallhydroxide oder -oxide, besonders bevorzugt jedoch Natriumhydroxid, Natriumcarbonat oder Natriumhydrogencarbonat. Üblicherweise wird die Neutralisation durch Einmischung des Neutralisationsmittels als wäßrige Lösung oder bevorzugt auch als Feststoff erreicht. Das Gel wird hierzu mechanisch zerkleinert, beispielsweise mittels eines Fleischwolfes, und das Neutralisationsmittel wird aufgesprüht, übergestreut oder aufgegossen, und dann scrgfältig untergemischt. Dazu kann die erhaltene Gelmasse noch mehrmals zur Homogenisierung gewolft werden.

Die neutralisierte Gelmasse wird dann mit einem Band- oder Walzentrockner getrocknet bis der Restfeuchtegehalt unter 10 Gew.-%, bevorzugt unter 5 Gew.-% liegt. Das getrocknete Hydrogel wird hernach gemahlen und gesiebt, wobei zur Mahlung üblicherweise Walzenstühle, Stiftmühlen oder Schwingmühlen eingesetzt werden können. Die bevorzugte Partikelgröße des gesiebten Hydrogels liegt im Bereich 45-1000 µm, besonders bevorzugt bei 45-850 µm, und ganz besonders bevorzugt bei 200-850 µm.

Erfindungsgemäß werden zur Vernetzung von acrylathaltigen Polymeren Bis- oder Poly-2-oxazolidinone eingesetzt. Der erfindungsgemäße, neue Vernetzer läßt sich durch die Reaktion von Isocyanaten und Diepoxiden (Bis-2-Oxazolidinone) oder von Diisocyanaten mit Diepoxiden (Poly-2-oxazolidinone) herstellen.

Die allgemeine Reaktionsgleichung für die Bildung von Bis-2-oxazolidinonen lautet:

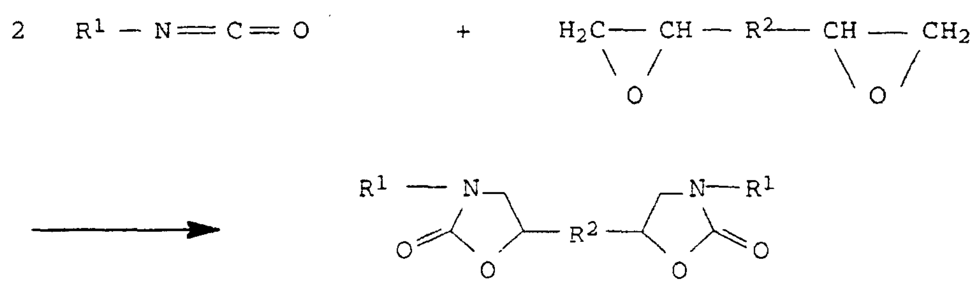

Die Bildung von Poly-2-oxazolidinonen kann durch die allgemeine Reaktionsgleichung für die Polyaddition beschrieben werden:

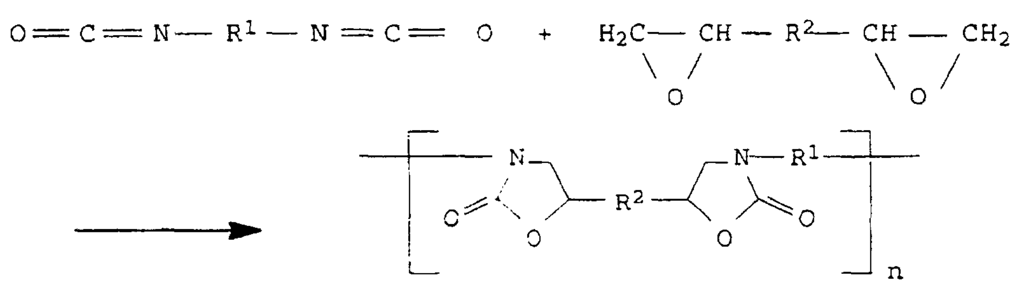

$R^1$ und $R^2$ haben die bereits erwahnte Bedeutung. Bei der Herstellung von Poly-2-oxazolidinonen kann dem Reaktionsgemisch eine gewisse Menge an Mono-Isocyanaten zugesetzt werden. Diese bewirken einen Abbruch der Polyaddition, wenn sie eingebaut werden. Die zugesetzte Menge an Mono-Isocyanaten muß dann so bemessen sein, daß sie zur gewünschten Kettenlänge der Poly-2-oxazolidinone führt. Der die Isocyanatgruppe tragende Rest ist dann die Endgruppe. In der Wiederholungseinheit des Polymeren haben sich durch die Polyadditionsreaktion zwei Oxazolidinoneinheiten gebildet wobei n ganzzahlig und größer als 1 ist. Diese Polyadditionsreaktion verläuft vorzugsweise, aber nicht ausschließlich, in polaren, aprotischen Lösungsmitteln, die weder mit den Diisocyanat noch dem Diepoxid bei der vorgegebenen Temperatur reagieren. Aus aliphatischen Diisocyanaten entstehen mit Diepoxiden helle, niedrigschmelzende Produkte, die z.B. in Dimethylformamid löslich sind. Dagegen ergibt die Reaktion von aromatischen Diisocyanaten meist dunkelgefärbte, weitgehend unlösliche und hochschmelzende Produkte.

Bei Verwendung aromatischer Diisocyanate ergeben sich meist hochschmelzende und schwerlösliche Produkte, daher wird die Verwendung von aliphatischen Diisocyanaten mit aliphatischen oder aromatischen Diepoxiden bevorzugt.

Ein weiterer Gegenstand der Erfindung ist ein Produkt hergestellt nach dem oben beschriebenen Verfahren.

Ein weiterer Gegenstand der Erfindung ist die Verwendung der nach dem erfindungsgemäßen Verfahren hergestellten Produkte in Hygenieartikeln, Verpackungsmaterialien, und in Nonwovens.

Zur Bestimmung der Güte der Oberflächennachvernetzung wird das getrocknete Hydrogel dann mit den im Stand der Technik bekannten, Testmethoden geprüft, die nachfolgend beschrieben sind:

Methoden:

1) Zentrifugenretentionskapazität (CRC):

Bei dieser Methode wird die freie Quellbarkeit des Hydrogels im Teebeutel bestimmt. Es werden ca. 0.200 g trockenes Hydrogel in einen Teebeutel eingeschweißt (Format: 60 mm x 60 mm, Dexter 1234T-Papier) und für 30 Minuten in einer 0,9 gew.-%ige Kochsalzlösung eingeweicht. Anschließend wird der Teebeutel 3 min in einer handelsüblichen Wäschezentrifuge (Bauknecht WS 130, 1400 U/min, Korbdurchmesser 230 mm) geschleudert. Die Bestimmung der aufgenommenen Flüssigkeitsmenge geschieht durch auswägen des zentrifugierten Teebeutels. Zur Berücksichtigung der Aufnahmekapazität des Teebeutels selbst wird ein Blindwert bestimmt (Teebeutel ohne Hydrogel), welcher von der Auswaage (Teebeutel mit gequollenem Hydrogel) abgezogen wird.
Retention CRC [g/g] = (Auswaage Teebeutel - Blindwert - Einwaage Hydrogel) ÷ Einwaage Hydrogel

2) Absorption unter Druck (0,3 / 0,5 / 0,7 psi):

Bei der Absorption unter Druck werden 0,900 g trockenen Hydrogels gleichmäßig auf dem Siebboden einer Meßzelle verteilt. Die Meßzelle besteht aus einem Plexiglaszylinder (Höhe = 50 mm, Durchmesser = 60 mm), auf den als Boden ein Sieb aus Stahlgewebe (Maschenweite 36 micron bzw. 400 mesh) aufgeklebt ist. Über das gleichmäßig verteilte Hydrogel wird eine Abdeckplatte gelegt und mit einem entsprechenden Gewicht belastet. Die Zelle wird dann auf ein Filterpapier (S&S 589 Schwarzband, Durchmesser = 90 mm) gestellt, welches auf einer porösen Glasfilterplatte liegt, diese Filterplatte liegt in einer Petrischale (Höhe = 30 mm, Durchmesser = 200 mm), welche soviel 0,9 gew.-%ige Kochsalzlösung enthält, daß der Flüssigkeitsspiegel zu Beginn des Experiments identisch mit der Oberkante der Glasfritte ist. Man läßt das Hydrogel dann für 60 min die Salzlösung absorbieren. Dann nimmt man die komplette Zelle mit dem gequollenen Gel von der Filterplatte, und wägt die Apparatur nach Entfernen des Gewichts zurück.
Die Absorption unter Druck (AUL = Absorbency under load) wird wie folgt berechnet:

$$AUL\ [g/g] = (Wb - Wa) / Ws$$

wobei Wb die Masse der Apparatur + Gel nach dem Quellen ist, Wa die Masse der Apparatur + Einwaage vor dem Quellen ist, Ws die Einwaage an trockenem Hydrogel ist.
Die Apparatur besteht aus Messzylinder und Abdeckplatte.

Beispiele

Die erfindungsgemäßen Beispiele zeigen den Effekt der Oberflächennachvernetzung auf die superabsorbierenden Polymere. Wie dem Fachmann bekannt ist, kann diese Nachvernetzung durch die Messung der Zentrifugenretention (CRC) und der Absorption under Belastung (AUL) bestimmt werden. Bei dieser Oberflächenvernetzung sinkt die CRC typischerweise um 5-10 g/g, während die AUL 0,7 psi um ungefähr 10, die AUL 0,3 psi um mehr auf 20 g/g zunimmt.

Beispiel 1

Grundpolymer

In einem 40 1-Plastikeimer werden 6,9 kg reine Acrylsäure mit 23 kg Wasser verdünnt. Zu dieser Lösung fügt man 45 g Pentaerythritoltriallylether unter Rühren hinzu, und inertisiert den verschlossenen Eimer durch Durchleiten von Stickstoff. Die Polymerisation wird dann durch Zugabe von ca. 400 mg Wasserstoffperoxid und 200 mg Ascorbinsäure gestartet. Nach Beendung der Reaktion wird das Gel mechanisch zerkleinert, und mit soviel Natronlauge versetzt bis ein Neutralisationsgrad von 75 mol-% bezogen auf die eingesetzte Acrylsäure erreicht wird. Das neutralisierte Gel wird dann auf einem Walzentrockner getrocknet, mit einer Stiftmühle gemahlen, und schließlich abgesiebt. Dies ist das in den nachfolgenden Beispielen verwendete Grundpolymer.

Beispiel 1a und 1b

Das Grundpolymer wird in einem Waring-Labormischer mit Vernetzerlösung folgender Zusammensetzung besprüht: 5 % Methanol, 5 % Wasser, 0,20 % Bisoxazolidinon aus Herstellbeispiel 1 - bezogen auf eingesetztes Polymer. Anschließend wird ein Teil des feuchten Produkts bei 170°C für 60 Minuten und der Rest bei 170°C für 90

Minuten im Umlufttrockenschrank getempert. Das getrocknete Produkt wird bei 850 micron abgesiebt, um Klumpen zu entfernen. Dies Beispiel zeigt, daß bei Verwendung von Bisoxazolidinonen keine Diolkomponente erforderlich ist, um eine Oberflächennachvernetzung zu erreichen.

Beispiel 2a und 2b

Grundpolymer gemäß Beispiel 1 hergestellt, wird in einem Waring-Labormischer mit Vernetzerlösung besprüht. Die Lösung ist dabei so zusammengesetzt, daß folgende Dosierung bezogen auf eingesetztes Grundpolymer erreicht wird: 0,20 Gew.-% Bisoxazolidinon aus Herstellbeispiel 2, 5 Gew.-% Propylenglykol, 5 Gew.-% Wasser. Das feuchte Polymer wird dann bei 175°C für 30 bzw. 60 min getrocknet.

Beispiel 3a und 3b

Grundpolymer gemäß Beispiel 1 hergestellt, wird in einem Waring-Labormischer mit Vernetzerlösung besprüht. Die Lösung ist dabei so zusammengesetzt, daß folgende Dosierung bezogen auf eingesetztes Grundpolymer erreicht wird: 0,20 Gew.-% Poly-2-oxazolidinon aus Herstellbeispiel 3, 5 Gew.-% Propylenglykol, 5 Gew.-% Wasser, und 0,2 Gew.-% Borsäure. Das feuchte Polymer wird dann bei 175°C für 60 bzw. 90 min getrocknet.

Beispiel 4a und 4b

Grundpolymer gemäß Beispiel 1 hergestellt, wird in einem Waring-Labormischer mit Vernetzerlösung besprüht. Die Lösung ist dabei so zusammengesetzt, daß folgende Dosierung bezogen auf eingesetz tes Grundpolymer erreicht wird: 0,20 Gew.-% Poly-2 oxazolidinon aus Herstellbeispiel 4, 5 Gew.-% Propylenglykol, Gew.-% Wasser, und 0,2 Gew.-% Ammoniumdihydrogenphosphat. Das feuchte Polymer wird dann bei 175°C für 60 bzw. 90 min getrocknet.

Beispiel 5a und 5b

Grundpolymer gemäß Beispiel 1 hergestellt, wird in einem Waring-Labormischer mit Vernetzerlösung besprüht. Die Lösung ist dabei so zusammengesetzt, daß folgende Dosierung bezogen auf eingesetztes Grundpolymer erreicht wird: 0,20 Gew.-% Poly-2-oxazolidinon aus Herstellbeispiel 5, 5 Gew.-% Propylenglykol, 5 Gew.-% Wasser, und 0,2 Gew.-% Ammoniumdihydrogenphosphat. Das feuchte Polymer wird dann bei 175°C für 60 bzw. 90 min getrocknet.

Beispiel 6a und 6b

Grundpolymer gemäß Beispiel 1 hergestellt wird in einem Waring-Labormischer mit Vernetzerlösung besprüht. Die Lösung ist dabei so zusammengesetzt, daß folgende Dosierung bezogen auf eingesetztes Grundpolymer erreicht wird: 0,20 Gew.-% Bisoxazolidinon aus Herstellbeispiel 2, 5 Gew.-% Methanol, 5 Gew.-% Wasser, und 0,2 Gew-% Ammoniumdihydrogenphosphat. Dieses Beispiel zeigt, daß bei Verwendung von Bisoxazolidinonen keine Diolkomponente erforderlich ist, um eine Oberflächennachvernetzung zu erreichen. Das feuchte Polymer wird dann bei 175°C für 60 bzw. 90 min getrocknet.

Beispiel 7a und 7b

Grundpolymer gemäß Beispiel 1 hergestellt, wird in einem Waring-Labormischer mit Vernetzerlösung besprüht. Die Lösung ist dabei so zusammengesetzt, daß folgende Dosierung bezogen auf eingesetztes Grundpolymer erreicht wird: 0,20 Gew.-% Poly-2-oxazolidinon aus Herstellbeispiel 5, 5 Gew.-% Methanol, 5 Gew.-% Wasser, und 0,2 Gew.-% Ammoniumdihydrogenphosphat. Dies Beispiel zeigt, daß bei Verwendung von Poly-2-oxazolidinonen keine Diolkomponente erforderlich ist, um eine Oberflächennachvernetzung zu erreichen. Das feuchte Polymer wird dann bei 175°C für 60 bzw. 90 min getrocknet.

Darstellung eines Bis-2-Oxazolidinons, Herstellbeispiel 1 Reaktionsprodukt aus Resorcinoldiglycidylether und Phenylisocyanat

In einem Dreihalskolben mit Rührer, Rückflußkühler und Gaseinleitungsrohr werden 0,1 mol Resorcinol-diglycidylether (ABCR) mit 0,2 mol Phenylisocyanat (Aldrich) unter Stickstoffatmosphäre tropfenweise gemischt und bis zum Sieden des DMF (Aldrich) erhitzt. Die Reaktionslösung wird noch 3 bis 4 Stunden unter Rückfluß gerührt und anschließend abgekühlt. Das Produkt wird mit Methanol gefällt und aus DMF/Methanol umkristallisiert.

Man erhält in hoher Ausbeute eine braun-gelbe Verbindung mit einem Schmelzpunkt von 202°C. Das IR Spektrum zeigt die typische Bande für Oxazolidinone bei 1750 $cm^{-1}$. Die Elementaranalyse ergibt: C: 67,2 %, H: 5,3

%, N: 6,0 %

Darstellung eines Bis-2-Oxazolidinons, Herstellbeispiel 2 Reaktionsprodukt aus Bisphenol-A-bisglycidylether und Phenylisocyanat

In einem Dreihalskolben mit Rührer, Rückflußkühler und Gaseinleitungsrohr wird 0,1 mol Bisphenol-A-diglycidylether (Aldrich) mit 0,2 mol Phenylisocyanat unter Stickstoffatmosphäre tropfenweise gemischt und bis zum Sieden des DMF erhitzt. Die Reaktionslösung wird noch 3-4 Stunden unter Rückfluß gerührt und anschließend abgekühlt. Das Produkt wird mit Methanol gefällt und aus Wasser/ Methanol (3:1) umkristallisiert. Man erhält in hoher Ausbeute eine braun-gelbe Verbindung mit einem Schmelzpunkt von 131°C. Das IR Spektrum zeigt die typische Bande für Oxazolidinone bei 1760 $cm^{-1}$. Die Elementaranalyse ergibt: C: 72,2 %, H: 5,4 %, N: 5,0 %

Darstellung von Poly-2-oxazolidinon, Herstellbeispiel 3

In einem Dreihalskolben mit Rührer, Rückflußkühler und Gaseinleitungsrohr wird 0,1 mol Bisphenol-A-diglycidylether mit 0,1 mol Hexamethylendiisocyanat unter Stickstoffatmosphäre tropfenweise gemischt und bis zum Sieden des DMF erhitzt. Die Reaktionslösung wird noch 3 bis 4 Stunden unter Rückfluß gerührt und anschließend abgekühlt. Das Produkt wird mit Wasser gefällt und erneut in DMF gelöst und mit Wasser ausgefällt. Das Produkt läßt sich in Methanol und Methanol/Wasser Mischungen lösen. Es entsteht ein weißer Feststoff mit einem Schmelzbereich zwischen 105 und 120°C und einem Staudingerindex in destilliertem Wasser $\eta_{sp}/c$ von 0,024 l/g.

Darstellung von Poly-2-oxazolidinon, Herstellbeispiel 4

In einem Dreihalskolben mit Rührer, Rückflußkühler und Gaseinleitungsrohr wird 0,1 mol Butandioldiglycylether = 1,4-Bis-(2,3epoxypropoxy)-butan mit 0,1 mol Hexamethylendiisocyanat unter Stickstoffatmosphäre tropfenweise gemischt und bis zum Sieden des DMF erhitzt. Die Reaktionslösung wird noch 3 bis 4 Stunden unter Rückfluß gerührt und anschließend abgekühlt. Das Produkt wird mit Methanol gefällt und aus Wasser/Methanol (3:1) umkristallisiert. Es ergibt sich eine hellgelbe, ölige Flüssigkeit, die mit Methanol und Methanol/Wasser mischbar ist.

Darstellung von Poly-2-oxazolidinon, Herstellbeispiel 5

In einem Dreihalskolben mit Rührer, Rückflußkühler und Gaseinleitungsrohr wird 0,1 mol Hexandioldiglycylether = 1,4-Bis-(2,3epoxypropoxy)-hexan (ABCR) mit 0,1 mol Hexamethylendiisocyanat unter Stickstoffatmosphäre tropfenweise gemischt und bis zum Sieden des DMF erhitzt. Die Reaktionslösung wird noch 3 bis 4 Stunden unter Rückfluß gerührt und anschließend abgekühlt. Das Produkt wird mit Methanol gefällt und aus Wasser/Methanol (3:1) umkristallisiert. Es ergibt sich eine hellgelbe, ölige Flüssigkeit, die mit Methanol und Methanol/Wasser mischbar ist.

Die anwendungstechnischen Daten der gemäß den Beispielen 1a, 1b - 7a, 7b hergestellten Polymere sind der nachfolgenden Tabelle zu entnehmen.

Trocknungstemperatur und -zeit beziehen sich hierbei auf die Temperung des mit Oberflächennachvernetzungslösung besprühten Grundpolymers.

Tabelle

| erfindungsgemäße Beispiele | vernetzt mit Produkt aus Herstellbeispiel | Trocknungstemperatur | Trocknungszeit | Katalysator/ Lösemittel | CRC [g/g] | AUL 0,3 psi [g/g] | AUL 0,7 psi [g/g] |
|---|---|---|---|---|---|---|---|
| Beispiel 1 - Grundpolymer | | -- | -- | -- | 42 | 10 | 9 |
| Beispiel 1a | 1 | 170°C | 60 min | kein Kat. MeOH / $H_2O$ | 35 | 36 | 21 |
| Beispiel 1b | 1 | 170°C | 90 min | kein Kat. MeOH / $H_2O$ | 32 | 34 | 27 |
| Beispiel 2a | 2 | 175°C | 30 min | kein Kat. PG /$H_2O$ | 35 | 35 | 27 |
| Beispiel 2b | 2 | 175°C | 60 min | kein Kat. PG / $H_2O$ | 34 | 33 | 25 |
| Beispiel 3a | 3 | 175°C | 60 min | 0,2 % $H_3BO_3$ PG /$H_2O$ | 32 | 35 | 25 |
| Beispiel 3b | 3 | 175°C | 90 min | 0,2 % $H_3BO_3$ PG /$H_2O$ | 33 | 36 | 26 |
| Beispiel 4a | 4 | 175°C | 60 min | $NH_4H_2PO_4$ PG /$H_2O$ | 34 | 34 | 24 |
| Beispiel 4b | 4 | 175°C | 90 min | $NH_4H_2PO_4$ PG /$H_2O$ | 32 | 35 | 25 |

| erfindungsgemäße Beispiele | vernetzt mit Produkt aus Herstellbeispiel | Trocknungstemperatur | Trocknungszeit | Katalysator/ Lösemittel | CRC [g/g] | AUL 0,3 psi [g/g] | AUL 0,7 psi [g/g] |
|---|---|---|---|---|---|---|---|
| Beispiel 5a | 5 | 175°C | 60 min | $NH_4H_2PO_4$ PG /$H_2O$ | 33 | 36 | 24 |
| Beispiel 5b | 5 | 175°C | 90 min | $NH_4H_2PO_4$ PG /$H_2O$ | 32 | 34 | 25 |
| Beispiel 6a | 2 | 175°C | 60 min | $NH_4H_2PO_4$ MeOH/ $H_2O$ | 32 | 29 | 20 |
| Beispiel 6b | 2 | 175°C | 90 min | $NH_4H_2PO_4$ MeOH/$H_2O$ | 30 | 30 | 20 |
| Beispiel 7a | 5 | 175°C | 60 min | $NH_4H_2PO_4$. MeOH/ $H_2O$ | 32 | 31 | 21 |
| Beispiel 7b | 5 | 175°C | 90 min | $NH_4H_2PO_4$ MeOH $H_2O$ | 31 | 30 | 19 |

## Patentansprüche

1. Verfahren zur Oberflächennachvernetzung wasserabsorbierender Polymere, **dadurch gekennzeichnet, daß** die Polymere mit einer Oberflächennachvernetzungslösung behandelt und während oder nach dem Behandeln durch Temperaturerhöhung nachvernetzt und getrocknet werden, **dadurch gekennzeichnet, daß** der Vernetzer ein Bis-2-Oxazolidinon oder ein Poly-2-Oxazolidinon, enthaltend Struktureinheiten der allgemeinen Formel 1

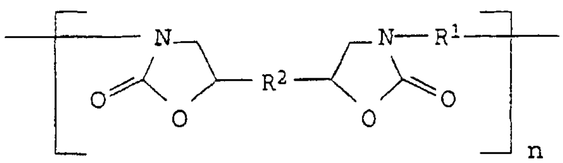

(1),

worin

$R^1$ verzweigtes oder unverzweigtes $C_1$-$C_{18}$-Alkylen, verzweigtes oder unverzweigtes $C_2$-$C_{18}$-Alkenylen, $C_5$-$C_8$-Cycloalkylen, Phenylen, Naphthylen, Anthracenylen, mit Kohlenwasserstoffen substituiertes Phenylen, Naphthylen oder Anthracenylen oder ein anderer substituierter oder unsubstituierter $C_6$-$C_{18}$-Arylenrest,

$R^2$ verzweigtes oder unverzweigtes $C_1$-$C_{18}$-Alkylen und

n eine ganze Zahl von 1 bis 50 bedeuten,

oder ein Gemisch von Bis-2-Oxazolidinonen und Poly-2-Oxazolidinonen gelöst in einem inerten Lösemittel enthält.

2. Verfahren nach Anspruch 1, **dadurch gekennzeichnet, daß** das wasserabsorbierende Polymer eine polymere Acrylsäure oder ein Polyacrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde, der zusätzlich noch mindestens eine freie Hydroxylgruppe trägt.

3. Verfahren nach Anspruch 1 oder 2, **dadurch gekennzeichnet, daß** zur Vernetzung ein Katalysator verwendet wird, der eine anorganische Säure, deren korrespondierendes Anhydrid, oder eine organische Säure oder deren korrespondierendes Anhydrid umfaßt.

4. Verfahren nach Anspruch 3, **dadurch gekennzeichnet, daß** es sich bei den Säuren um Borsäure, Schwefelsäure, Iodwasserstoffsäure, Phosphorsäure, Weinsäure, Essigsäure, Toluolsulfonsäure, sowie deren polymere Formen, Anhydride oder sauren Salze handelt.

5. Verfahren nach einem oder mehreren der Ansprüche 1 bis 4, **dadurch gekennzeichnet, daß** das inerte Lösemittel Wasser, ein Gemisch von Wasser mit in Wasser unbegrenzt löslichen organischen Lösemitteln oder ein Gemisch von Wasser mit einfachen oder mehrfachfunktionellen Alkoholen ist.

6. Verfahren nach Anspruch 5, **dadurch gekennzeichnet, daß** bei Verwendung eines Alkohol/Wasser-Gemischs der Alkoholgehalt dieser Lösung 10 - 90 Gew.-%, bevorzugt 30 - 70 Gew.-% beträgt.

7. Verfahren nach Anspruch 5 oder 6, **dadurch gekennzeichnet, daß** der Alkohol Methanol, Ethanol, Isopropanol, Ethylenglykol, 1,2-Propandiol oder 1,3-Propandiol ist.

8. Verfahren nach einem oder mehreren der Ansprüche 1 bis 4, **dadurch gekennzeichnet, daß** die Oberflächennachvernetzungslösung in einem Verhältnis von 1-20 Gew.-%, insbesondere 2,5-15 Gew.-% bezogen auf die Masse des Polymeren eingesetzt wird.

9. Flüssigkeitsabsorbierende Polymere, hergestellt nach dem Verfahren nach einem oder mehreren der Ansprüche 1 bis 8.

10. Verwendung der nach dem Verfahren nach einem oder mehreren der Ansprüche 1 bis 8 hergestellten Polymere

in Hygieneartikeln, Verpackungsmaterialien und in Nonwovens.

## Claims

1. A process for the surface postcrosslinking of water-absorbing polymers, wherein the polymers are treated with a surface postcrosslinking solution and during or after the treatment are postcrosslinked by means of an increase in temperature and are dried, wherein the crosslinker comprises a bis-2-oxazolidinone or a poly-2-oxazolidinone comprising structural units of the formula 1

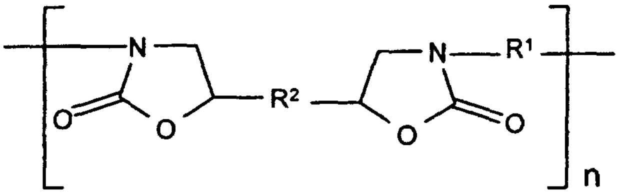

(1)

in which
$R^1$ is branched or unbranched $C_1$-$C_{18}$-alkylene, branched or unbranched $C_2$-$C_{18}$-alkenylene, $C_5$-$C_8$-cycloalkylene, phenylene, naphthylene, anthracenylene, hydrocarbon-substituted phenylene, naphthylene or anthracenylene or another substituted or unsubstituted $C_6$-$C_{18}$-arylene radical,
$R^2$ is branched or unbranched $C_1$-$C_{18}$-alkylene and
n is an integer from 1 to 50
or a mixture of bis-2-oxazolidinones and poly-2-oxazolidinones dissolved in an inert solvent.

2. The process as claimed in claim 1, wherein the water-absorbing polymer is a polymeric acrylic acid or a polyacrylate, especially a polymeric acrylic acid or a polyacrylate obtained by means of free-radical addition polymerization using a polyfunctional ethylenically unsaturated free-radical crosslinker which in addition carries one or more free hydroxyl groups.

3. The process as claimed in claim 1 or 2, wherein a catalyst is used for crosslinking which comprises an inorganic acid, its corresponding anhydride, or an organic acid or its corresponding anhydride.

4. The process as claimed in claim 3, wherein the acids are boric, sulfuric, hydroiodic, phosphoric, tartaric, acetic or toluenesulfonic acid or their polymeric forms, anhydrides or acid salts.

5. The process as claimed in one or more of claims 1 to 4, wherein the inert solvent is water, a mixture of water with organic solvents of unlimited solubility in water, or a mixture of water with simple or polyfunctional alcohols.

6. The process as claimed in claim 5, wherein if an alcohol/water mixture is used the alcohol content of this solution is 10 - 90% by weight, preferably 30 - 70% by weight.

7. The process as claimed in claim 5 or 6, wherein the alcohol is methanol, ethanol, isopropanol, ethylene glycol, 1,2-propanediol or 1,3-propanediol.

8. The process as claimed in one or more of claims 1 to 4, wherein the surface postcrosslinking solution is employed in a proportion of 1 - 20% by weight, in particular 2.5 - 15% by weight, based on the mass of the polymer.

9. A liquid-absorbing polymer prepared by the process as claimed in one or more of claims 1 to 8.

10. The use of the polymer prepared by the process as claimed in one or more of claims 1 to 8 in a hygiene article, packaging material or nonwoven.

## Revendications

1. Procédé de postréticulation superficielle de polymères absorbant l'eau, **caractérisé en ce que** les polymères sont traités par une solution de postréticulation superficielle et sont postréticulés et séchés pendant ou après le traitement par une élévation de température, et **en ce que** l'agent de réticulation contient une bis-2-oxazolidinone ou une poly-2-oxazolidinone, contenant des unités de structure de la formule générale 1 :

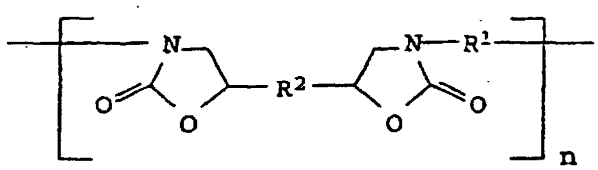

(1)

dans laquelle

$R^1$ représente de l'alkylène en $C_1$-$C_{18}$ ramifié ou non ramifié, de l'alcénylène en $C_2$-$C_{18}$ ramifié ou non ramifié, du cycloalkylène en $C_5$-$C_8$, du phénylène, du naphtylène, de l'anthracénylène, du phénylène, naphtylène ou anthracénylène substitué par des hydrocarbures ou un autre radical arylène en $C_6$-$C_{18}$ substitué ou non substitué,

$R^2$ représente de l'alkylène en $C_1$-$C_{18}$ ramifié ou non ramifié, et

n est un nombre entier de 1 à 50,

ou un mélange de bis-2-oxazolidinones et de poly-2-oxazolidinones, en solution dans un solvant inerte.

2. Procédé suivant la revendication 1, **caractérisé en ce que** le polymère absorbant l'eau est un acide acrylique polymère ou un polyacrylate, en particulier un acide acrylique polymère ou un polyacrylate qui a été obtenu par polymérisation radicalaire ou pour lequel on a utilisé un agent de réticulation radicalaire éthyléniquement insaturé, plurifonctionnel, qui porte en supplément encore au moins un groupe hydroxyle libre.

3. Procédé suivant l'une des revendications 1 et 2, **caractérisé en ce que**, pour la réticulation, on utilise un catalyseur qui comporte un acide inorganique, son anhydride correspondant, ou un acide organique ou son anhydride correspondant.

4. Procédé suivant la revendication 3, **caractérisé en ce que**, pour ce qui concerne les acides, il s'agit d'acide borique, d'acide sulfurique, d'acide iodhydrique, d'acide phosphorique, d'acide tartrique, d'acide acétique, d'acide toluènesulfonique, ainsi que de leurs formes polymères, de leurs anhydrides ou de leurs sels acides.

5. Procédé suivant une ou plusieurs des revendications 1 à 4, **caractérisé en ce que** le solvant inerte est de l'eau, un mélange d'eau avec des solvants organiques solubles de manière illimitée dans l'eau ou un mélange d'eau avec des alcools à simple ou multiple fonction.

6. Procédé suivant la revendication 5, **caractérisé en ce que**, lors de l'utilisation d'un mélange d'alcool/eau, la teneur en alcool de cette solution s'élève à 10-90% en poids, de préférence de 30 à 70% en poids.

7. Procédé suivant l'une des revendications 5 et 6, **caractérisé en ce que** l'alcool est du méthanol, de l'éthanol, de l'isopropanol, de l'éthylèneglycol, du 1,2-propanediol ou du 1,3-propanediol.

8. Procédé suivant une ou plusieurs des revendications 1 à 4, **caractérisé en ce que** la solution de postréticulation superficielle est mise en oeuvre dans des proportions de 1-20% en poids, en particulier de 2,5-15% en poids, par rapport à la masse du polymère.

9. Polymères absorbant du liquide, préparés selon le procédé suivant une ou plusieurs des revendications 1 à 8.

10. Utilisation des polymères préparés suivant le procédé selon une ou plusieurs des revendications 1 à 8, dans des articles d'hygiène, des matériaux d'emballage et dans des non-tissés.